# EUROPÄISCHE PATENTSCHRIFT

(11) **EP 0 694 519 B1**
(45) Veröffentlichungstag und Bekanntmachung des Hinweises auf die Patenterteilung: **16.06.1999**
(21) Anmeldenummer: 95111065.9
(22) Anmeldetag: 14.07.1995
(51) Int. Cl.: C07C 45/46, C07C 49/807

(54) **Verfahren zur Herstellung von 2,4-Difluoracetophenon**
Process for the preparation of 2,4-difluoroacetophenone
Procédé pour la préparation de 2,4-difluoroacétophénone

(30) Priorität: 27.07.1994 DE 4426575
(43) Veröffentlichungstag der Anmeldung: 31.01.1996
(73) Patentinhaber: Clariant GmbH, 65929 Frankfurt am Main (DE)
(72) Erfinder: Meier, Michael, Dr., D-60487 Frankfurt am Main (DE); Pressler, Wilfried, Dr., D-65779 Kelkheim (DE)

(56) Entgegenhaltungen:
- FR-A- 1 569 165

## Beschreibung

Die Erfindung betrifft ein Verfahren zur Herstellung von 2,4-Difluoracetophenon in hohen Ausbeuten durch Umsetzung von 1,3-Difluorbenzol mit Acetylchlorid bzw. Acetanhydrid in Gegenwart von einem Friedel-Crafts-Katalysator.

2,4-Difluoracetophenon ist ein wichtiges Zwischenprodukt für die Herstellung von Pharmazeutika und Pflanzenschutzmitteln.

Es ist bekannt, daß man 2,4-Difluoracetophenon durch Umsetzung von 1 mol 1,3-Difluorbenzol mit Acetanhydrid bzw. Acetylchlorid unter Zusatz von Aluminiumchlorid in Schwefelkohlenstoff herstellen kann. (J.Chem.Soc. Perkin Trans 2 1974, 119 und Izv. Akad. Nauk SSR Ser. Khim 1959, 58 (eng. Ausgabe)). Die besten Ausbeuten betragen bei einem Einsatzverhältnis von 1 mol 1,3-Difluorbenzol, 1 mol Acetylchlorid und 1,5 mol Aluminiumchlorid 80 - 85 % d.Th.. Außerdem ist die Umsetzung von 1,3-Difluorbenzol mit Acetylchlorid und Aluminiumchlorid in 1,2-Dichlorethan bekannt (US 4 613 611). Eine Ausbeute wird nicht angegeben. Nachteilig an diesen Verfahren ist, daß toxikologisch und ökologisch bedenkliche Lösungsmittel eingesetzt werden.

In der GB-A-1-135 358 wird ein Verfahren zur Herstellung von 2,4-Dihalogeno-acetophenon durch Acetylierung von 1,3-Dihalogeno-benzol in Gegenwart eines Friedel-Crafts-Katalysators und in Gegenwart von 1,4-Dihalogeno-benzol, das an der Reaktion nicht teilnimmt, beschrieben.

Es bestand daher ein Bedürfnis nach einem wirtschaftlichen und technisch leicht durchführbaren Verfahren zur Herstellung von 2,4-Difluoracetophenon ohne den Einsatz von toxikologisch und ökologisch bedenklichen Lösungsmitteln.

Diese Aufgabe wird gelöst durch ein Verfahren zur Herstellung von 2,4-Difluoracetophenon, dadurch gekennzeichnet, daß man 1,3-Difluorbenzol mit einem Acetylierungsmittel in Gegenwart von einem Friedel-Crafts-Katalysator in überschüssigem 1,3-Difluorbenzol als Lösungsmittel und in Abwesenheit eines zusätzlichen Lösungsmittels umsetzt.

Als Acetylierungsmittel werden vorzugsweise Acetanhydrid und Acetylchlorid eingesetzt.

Als Friedel-Crafts-Katalysatoren werden insbesondere Aluminiumchlorid und Fluoralkansulfonsäure wie z.B. Hexafluorpropansäure oder Nonafluorbutansulfonsäure, bevorzugt Aluminiumchlorid eingesetzt.

Üblicherweise verfährt man hierbei so, daß man zu einer Suspension bzw. Lösung von Friedel-Crafts-Katalysator in überschüssigem 1,3-Difluorbenzol das Acetylierungsmittel zudosiert. Man kann aber auch so vorgehen, daß man das Acetylierungsmittel in überschüssigem 1,3-Difluorbenzol vorlegt und den Friedel-Crafts-Katalysator zudosiert.

1,3-Difluorbenzol wird in einer Menge von 1,2 bis 5,0 mol, vorzugsweise 1,8 bis 3,0 mol pro mol Acetylierungsmittel eingesetzt. Das überschüssige 1,3-Difluorbenzol kann nach Abschluß der Reaktion durch Destillation zurückgewonnen werden.

Aluminiumchlorid wird in einer Menge von 0,9 bis 1,8 mol, bevorzugt 1,1 bis 1,5 mol pro mol Acetylchlorid bzw. von 1,8 bis 3,6 mol, bevorzugt 2,2 bis 3,0 mol pro mol Acetanhydrid eingesetzt. Fluoralkansulfonsäuren werden in einer Menge von 0,05 bis 1,0 mol, bevorzugt 0,1 bis 0,5 mol pro mol Acetylierungsmittel eingesetzt.

Die Reaktion von 1,3-Difluorbenzol mit Acetylchlorid bzw. Acetanhydrid und Aluminiumchlorid als Katalysator wird bei Temperaturen von 10 bis 82°C, bevorzugt 25 bis 55°C durchgeführt. Die Reaktion von 1,3-Difluorbenzol mit Acetanhydrid unter Zusatz von Fluoralkansulfonsäuren wird bei 82 bis 150°C unter Druck durchgeführt.

Zur Aufarbeitung wird das Reaktionsgemisch hydrolisiert, die organische Phase abgetrennt, azeotrop getrocknet und destilliert.
Das zurückgewonnene 1,3-Difluorbenzol kann in einem Folgeansatz wieder eingesetzt werden.

Der Vorteil des erfindungsgemäßen Verfahrens ist, daß keine zusätzlichen toxikologisch und ökologisch bedenklichen Lösungsmittel verwendet werden. 2,4-Difluoracetophenon entsteht in Ausbeuten von ca. 94 %. Dies ist deutlich höher als der Stand der Technik. Durch die Rückführung des als Lösungsmittel eingesetzten 1,3-Difluorbenzol wird das Verfahren besonders wirtschaftlich.

### Beispiel 1

Zu 297 g 1,3-Difluorbenzol werden 155,6 g (1,17 mol) Aluminiumchlorid zudosiert und bei 35°C über 3 h 78,5 g (1,0 mol) Acetylchlorid zudosiert. Anschließend wird 1 h bei dieser Temperatur nachgerührt, auf Raumtemperatur abgekühlt und das Reaktionsgemisch auf eine Mischung von 200 g Eis und 200 g Wasser getropft. Die organische Phase wird abgetrennt und die wäßrige Phase mit 40 g 1,3-Difluorbenzol gewaschen. Die vereinigten organischen Phasen wurden zweimal mit insgesamt 200 g Wasser gewaschen und anschließend am Wasserabscheider Wasser ausgekreist. Die trockene organische Phase wurde destilliert. Bei einem Übergang von 82°C bis 108°C/100 mbar wurden 225 g 1,3-Difluorbenzol zurückgewonnen, die in einem Folgeansatz wieder eingesetzt werden. Bei 102 bis 106°C/80 mbar gehen 147,7 g 2,4-Difluoracetophenon über. Dies entspricht einer Ausbeute von 94,6 % d.Th..

### Beispiel 2

Zu 225 g zurückgewonnenem 1,3-Difluorbenzol und 65 g frischem 1,3-Difluorbenzol werden 155,6 g (1,17 mol) Aluminiumchlorid zudosiert und bei 35°C über 3 h 78,5 g (1,0 mol) Acetylchlorid zudosiert. Die Aufarbeitung erfolgte wie in Beispiel 1 angegeben. Es werden 148,5 g 2,4-Difluoracetophenon erhalten, dies entspricht einer Ausbeute von 95,1 % d.Th..

### Beispiel 3

Zu 150 g 1,3-Difluorbenzol werden 165,0 g (1,24 mol) Aluminiumchlorid zudosiert und bei 35°C über 3 h 51,0 g (0,5 mol) Acetanhydrid zudosiert. Zur Aufarbeitung wird auf 300 g Eis und 300 g Wasser gegeben und dann wie unter Beispiel 1 beschrieben fortgefahren. Es werden 72,8 g 2,4-Difluoracetophenon erhalten; dies entspricht einer Ausbeute von 93,3 % d.Th..

## Patentansprüche

1. Verfahren zur Herstellung von 2,4-Difluoracetophenon, dadurch gekennzeichnet, daß man 1,3-Difluorbenzol mit einem Acetylierungsmittel in Gegenwart von einem Friedel-Crafts-Katalysator in überschüssigem 1,3-Difluorbenzol als Lösungsmittel und in Abwesenheit eines zusätzlichen Lösungsmittels umsetzt.

2. Verfahren nach Anspruch 1, dadurch gekennzeichnet, daß man als Acetylierungsmittel Acetylchlorid verwendet.

3. Verfahren nach Anspruch 1, dadurch gekennzeichnet, daß man als Acetylierungsmittel Acetanhydrid verwendet.

4. Verfahren nach mindestens einem der Ansprüche 1 bis 3, dadurch gekennzeichnet, daß man als Friedel-Crafts-Katalysator Aluminiumchlorid verwendet.

5. Verfahren nach mindestens einem der Ansprüche 1 bis 3, dadurch gekennzeichnet, daß man als Friedel-Crafts-Katalysatoren eine Fluoralkansulfonsäure verwendet.

6. Verfahren nach mindestens einem der Ansprüche 1 bis 5, dadurch gekennzeichnet, daß 1,3-Difluorbenzol in einer Menge von 1,2 bis 5,0 mol pro mol Acetylierungsmittel eingesetzt wird.

7. Verfahren nach mindestens einem der Ansprüche 1 bis 5, dadurch gekennzeichnet, daß 1,3-Difluorbenzol in einer Menge von 1,8 bis 3,0 mol pro mol Acetylierungsmittel eingesetzt wird.

8. Verfahren nach mindestens einem der Ansprüche 2, 4, 6 und 7, dadurch gekennzeichnet, daß man Aluminiumchlorid in einer Menge von 0,9 bis 1,8 mol, bevorzugt 1,1 bis 1,5 mol pro mol Acetylchlorid einsetzt.

9. Verfahren nach mindestens einem der Ansprüche 3, 4, 6 und 7, dadurch gekennzeichnet, daß man Aluminiumchlorid in einer Menge von 1,8 bis 3,6 mol, bevorzugt 2,2 bis 3,0 mol pro mol Acetanhydrid einsetzt.

10. Verfahren nach mindestens einem der Ansprüche 1 bis 3 und 5 bis 7, dadurch gekennzeichnet, daß man Fluoralkansulfonsäuren in einer Menge von 0,05 bis 1,0 mol pro mol Acetylierungsmittel einsetzt.

11. Verfahren nach mindestens einem der Ansprüche 1 bis 4 und 6 bis 9, dadurch gekennzeichnet, daß man 1,3-Difluorbenzol und Aluminiumchlorid mit einem Acetylierungsmittel bei Temperaturen von 10 bis 82°C, insbesondere 25 bis 55°C umsetzt.

12. Verfahren nach mindestens einem der Ansprüche 1 bis 3, 5 bis 7 und 10, dadurch gekennzeichnet, daß man 1,3-Difluorbenzol und Fluoralkansulfonsäure mit einem Acetylierungsmittel bei Temperaturen von 82 bis 150°C unter Druck umsetzt.

13. Verfahren nach mindestens einem der Ansprüche 1 bis 12, dadurch gekennzeichnet, daß man wiedergewonnenes 1,3-Difluorbenzol in die Reaktion einsetzt.

## Claims

1. A process for the preparation of 2,4-difluoroacetophenone, which comprises reacting 1,3-difluorobenzene with an acetylating agent in the presence of a Friedel-Crafts catalyst in excess 1,3-difluorobenzene as solvent and in the absence of an additional solvent.

2. The process as claimed in claim 1, wherein acetyl chloride is used as acetylating agent.

3. The process as claimed in claim 1, wherein acetic anhydride is used as acetylating agent.

4. The process as claimed in at least one of claims 1 to 3, wherein aluminum chloride is used as Friedel-Crafts catalyst.

5. The process as claimed in at least one of claims 1 to 3, wherein a fluoroalkanesulfonic acid is used as Friedel-Crafts catalyst.

6. The process as claimed in at least one of claims 1 to 5, wherein 1,3-difluorobenzene is employed in a quantity of from 1.2 to 5.0 mol per mole of acetylating agent.

7. The process as claimed in at least one of claims 1 to 5, wherein 1,3-difluorobenzene is employed in a quantity of from 1.8 to 3.0 mol per mole of acetylating agent.

8. The process as claimed in at least one of claims 2, 4, 6 and 7, wherein aluminum chloride is employed in a quantity of from 0.9 to 1.8 mol, preferably from 1.1 to 1.5 mol, per mole of acetyl chloride.

9. The process as claimed in at least one of claims 3, 4, 6 and 7, wherein aluminum chloride is employed in a quantity of from 1.8 to 3.6 mol, preferably from 2.2 to 3.0 mol, per mole of acetic anhydride.

10. The process as claimed in at least one of claims 1 to 3 and 5 to 7, wherein fluoroalkanesulfonic acids are employed in a quantity of from 0.05 to 1.0 mol per mole of acetylating agent.

11. The process as claimed in at least one of claims 1 to 4 and 6 to 9, wherein 1,3-difluorobenzene and aluminum chloride are reacted with an acetylating agent at temperatures of from 10 to 82°C, in particular from 25 to 55°C.

12. The process as claimed in at least one of claims 1 to 3, 5 to 7 and 10, wherein 1,3-difluorobenzene and fluoroalkanesulfonic acid are reacted with an acetylating agent under pressure at temperatures of from 82 to 150°C.

13. The process as claimed in at least one of claims 1 to 12, wherein recovered 1,3-difluorobenzene is employed in the reaction.

## Revendications

1. Procédé de préparation de 2,4-difluoroacétophénone, caractérisé en ce qu'on met à réagir du 1,3-difluorobenzène avec un agent d'acétylation en présence d'un catalyseur de Friedel et Crafts dans le 1,3-difluorobenzène en excès comme solvant et en l'absence d'un solvant additionnel.

2. Procédé selon la revendication 1, caractérisé en ce qu'on utilise le chlorure d'acétyle comme agent d'acétylation.

3. Procédé selon la revendication 1, caractérisé en ce qu'on utilise l'anhydride acétique comme agent d'acétylation.

4. Procédé selon au moins une des revendications 1 à 3, caractérisé en ce qu'on utilise le chlorure d'aluminium comme catalyseur de Friedel et Crafts.

5. Procédé selon au moins une des revendications 1 à 3, caractérisé en ce qu'on utilise comme catalyseurs de Friedel et Crafts un acide fluoroalcanesulfonique.

6. Procédé selon au moins une des revendications 1 à 5, caractérisé en ce qu'on utilise le 1,3-difluorobenzène en une quantité de 1,2 à 5,0 mol par mol d'agent d'acétylation.

7. Procédé selon au moins une des revendications 1 à 5, caractérisé en ce qu'on utilise le 1,3-difluorobenzène en une quantité de 1,8 à 3,0 mol par mol d'agent d'acétylation.

8. Procédé selon au moins une des revendications 2, 4, 6 et 7, caractérisé en ce qu'on utilise le chlorure d'aluminium en une quantité de 0,9 à 1,8 mol, de préférence de 1,1 à 1,5 mol par mole de chlorure d'acétyle.

9. Procédé selon au moins une des revendications 3, 4, 6 et 7, caractérisé en ce qu'on utilise le chlorure d'aluminium en une quantité de 1,8 à 3,6 mol, de préférence de 2,2 à 3,0 mol par mole d'anhydride acétique.

10. Procédé selon au moins une des revendications 1 à 3, et 5 à 7, caractérisé en ce qu'on utilise des acides fluoroalcanesulfoniques en une quantité de 0,05 à 1,0 mol par mole d'agent d'acétylation.

11. Procédé selon au moins une des revendications 1 à 4, et 6 à 9, caractérisé en ce qu'on met à réagir du 1,3-difluorobenzène et du chlorure d'aluminium avec un agent d'acétylation avec un agent d'acétylation à des températures de 10 à 82°C, en particulier de 25 à 55°C.

12. Procédé selon au moins une des revendications 1 à 3, 5 à 7 et 10, caractérisé en ce qu'on met à réagir du 1,3-difluorobenzène et un acide fluoroalcanesulfonique avec un agent d'acétylation à des températures de 82 à 150°C sous pression.

13. Procédé selon au moins une des revendications 1 à 12, caractérisé en ce qu'on utilise dans la réaction du 1,3-difluorobenzène récupéré.
